# EUROPEAN PATENT APPLICATION

(11) **EP 0 829 488 A1**
(43) Date of publication of application: **18.03.1998**
(21) Application number: 95917489.7
(22) Date of filing: 28.04.1995
(51) Int. Cl.: C07K 14/18, C07K 7/06, C07K 7/08, C07K 1/10, C07K 1/13, G01N 33/576

(54) **ANTIGEN PEPTIDE COMPOUND AND IMMUNOASSAY METHOD**

(71) Applicant: SRL, INC., Tachikawa-shi, Tokyo 190 (JP)
(72) Inventor: KUMAZAWA, Toshiaki Hachioji Laboratories, Tokyo 192 (JP); KIYA, Yoshiyasu Hachioji Laboratories, Tokyo 192 (JP)
(74) Representative: Herrmann-Trentepohl, Werner, Dipl.-Ing.
(86) International application number: JP9500849
(87) International publication number: WO9634013

(57) **Abstract**

A method of immunoassay of a hepatitis C virus (HCV) antigen in a specimen by utilizing the specific avidity of an antigen peptide compound having an amino acid sequence represented by the following formula (1) to (2) with a HCV antibody: (1) Arg-Lys-Thr-Ser-Glu-Arg-Ser-Gln-Pro-Arg-Gly-Arg-Arg-Gln-Pro-Ile-Pro-Lys-Ala-Arg-Arg-Pro-Glu-Gly-Arg-Thr-Trp-Ala-Gln-Pro ..., (2) Arg-Lys-Thr-Ser-Glu-Arg-Ser-Gln-Pro-Arg-Gly-Arg-Arg-Gln-Pro-Ile-Pro-Lys-Asp-Arg-Arg-Ser-Thr-Gly-Lys-Ser-Trp-Gly-Lys-Pro ... This method enables the serotype of a specimen to be determined readily and accurately while suppressing the cross reaction or nonspecific reaction, the effect of interferon therapy to be anticipated accurately, and the healing or the progress of treatment of hepatitis C to be observed in a simplified manner.

SEQUENCE LISTING

(1) GENERAL INFORMATION:

(i) APPLICANT:
(A) NAME: SRL, Inc.
(B) STREET: 41-19, Akebono-cho 2-chome
(C) CITY: Tachikawa-shi
(D) STATE: Tokyo
(E) COUNTRY: Japan
(F) POSTAL CODE (ZIP): 190

(ii) TITLE OF INVENTION: Antigenic Peptide Compound and Immunoassay

(iii) NUMBER OF SEQUENCES: 6

(iv) COMPUTER READABLE FORM:
(A) MEDIUM TYPE: Floppy disk
(B) COMPUTER: IBM PC compatible
(C) OPERATING SYSTEM: PC-DOS/MS-DOS
(D) SOFTWARE: PatentIn Release #1.0, Version #1.30 (EPO)

(v) CURRENT APPLICATION DATA:
APPLICATION NUMBER: EP 95917489.7
(vi) PRIOR APPLICATION DATA:
(A) APPLICATION NUMBER: WO PCT/JP95/00849
(B) FILING DATE: 28-APR-1995


(2) INFORMATION FOR SEQ ID NO: 1:

(i) SEQUENCE CHARACTERISTICS:
(A) LENGTH: 30 amino acids
(B) TYPE: amino acid
(C) STRANDEDNESS:
(D) TOPOLOGY: linear

(ii) MOLECULE TYPE: peptide

(iii) HYPOTHETICAL: NO

(v) FRAGMENT TYPE: internal

(vi) ORIGINAL SOURCE:
(A) ORGANISM: Hepatitis virus

(ix) FEATURE:
(A) NAME/KEY: Peptide
(B) LOCATION:6..30

(x) PUBLICATION INFORMATION:
(H) DOCUMENT NUMBER: WO 96/34013 A1
(I) FILING DATE: 28-APR-1995
(J) PUBLICATION DATE: 31-OCT-1996

(xi) SEQUENCE DESCRIPTION: SEQ ID NO: 1:

Arg Lys Thr Ser Glu Arg Ser Gln Pro Arg Gly Arg Arg Gln Pro Ile
1               5                   10                  15

Pro Lys Ala Arg Arg Pro Glu Gly Arg Thr Trp Ala Gln Pro
            20                  25                  30

(2) INFORMATION FOR SEQ ID NO: 2:

(i) SEQUENCE CHARACTERISTICS:
(A) LENGTH: 30 amino acids
(B) TYPE: amino acid
(C) STRANDEDNESS:
(D) TOPOLOGY: linear

(ii) MOLECULE TYPE: peptide

(iii) HYPOTHETICAL: NO

(v) FRAGMENT TYPE: internal

(vi) ORIGINAL SOURCE:
(A) ORGANISM: Hepatitis virus

(ix) FEATURE:
(A) NAME/KEY: Peptide
(B) LOCATION:6..30

(x) PUBLICATION INFORMATION:
(H) DOCUMENT NUMBER: WO 96/34013 A1
(I) FILING DATE: 28-APR-1995
(J) PUBLICATION DATE: 31-OCT-1996

(xi) SEQUENCE DESCRIPTION: SEQ ID NO: 2:

Arg Lys Thr Ser Glu Arg Ser Gln Pro Arg Gly Arg Arg Gln Pro Ile
1               5                   10                  15

Pro Lys Asp Arg Arg Ser Thr Gly Lys Ser Trp Gly Lys Pro
            20                  25                  30

(2) INFORMATION FOR SEQ ID NO: 3:

(i) SEQUENCE CHARACTERISTICS:
(A) LENGTH: 40 amino acids
(B) TYPE: amino acid
(C) STRANDEDNESS:
(D) TOPOLOGY: linear

(ii) MOLECULE TYPE: peptide

(iii) HYPOTHETICAL: NO

(v) FRAGMENT TYPE: internal

(vi) ORIGINAL SOURCE:
(A) ORGANISM: Hepatitis virus

(ix) FEATURE:
(A) NAME/KEY: Peptide
(B) LOCATION:1..30

(x) PUBLICATION INFORMATION:
(H) DOCUMENT NUMBER: WO 96/34013 A1
(I) FILING DATE: 28-APR-1995
(J) PUBLICATION DATE: 31-OCT-1996

(xi) SEQUENCE DESCRIPTION: SEQ ID NO: 3:

Ile Ile Leu Ser Gly Arg Pro Ala Ile Val Pro Asp Arg Glu Leu Leu
1               5                   10                  15

Tyr Gln Glu Phe Asp Glu Met Glu Glu Cys Ala Ser His Leu Pro Tyr
            20                  25                  30

Ile Glu Gln Gly Met Gln Leu Ala
        35                  40

(2) INFORMATION FOR SEQ ID NO: 4:

(i) SEQUENCE CHARACTERISTICS:
(A) LENGTH: 40 amino acids
(B) TYPE: amino acid
(C) STRANDEDNESS:
(D) TOPOLOGY: linear

(ii) MOLECULE TYPE: peptide

(iii) HYPOTHETICAL: NO

(v) FRAGMENT TYPE: internal

(vi) ORIGINAL SOURCE:
(A) ORGANISM: Hepatitis virus

(ix) FEATURE:
(A) NAME/KEY: Peptide
(B) LOCATION:1..30

(x) PUBLICATION INFORMATION:
(H) DOCUMENT NUMBER: WO 96/34013 A1
(I) FILING DATE: 28-APR-1995
(J) PUBLICATION DATE: 31-OCT-1996

(xi) SEQUENCE DESCRIPTION: SEQ ID NO: 4:

Leu His Val Asn Gln Arg Ala Val Val Ala Pro Asp Lys Glu Val Leu
1               5                   10                  15

Tyr Glu Ala Phe Asp Glu Met Glu Glu Cys Ala Ser Arg Ala Ala Leu
            20                  25                  30

Ile Glu Glu Gly Gln Arg Ile Ala
        35                  40

(2) INFORMATION FOR SEQ ID NO: 5:

(i) SEQUENCE CHARACTERISTICS:
(A) LENGTH: 20 amino acids
(B) TYPE: amino acid
(C) STRANDEDNESS:
(D) TOPOLOGY: linear

(ii) MOLECULE TYPE: peptide

(iii) HYPOTHETICAL: NO

(v) FRAGMENT TYPE: internal

(vi) ORIGINAL SOURCE:
(A) ORGANISM: Hepatitis virus

(ix) FEATURE:
(A) NAME/KEY: Peptide
(B) LOCATION:6..20

(x) PUBLICATION INFORMATION:
(H) DOCUMENT NUMBER: WO 96/34013 A1
(I) FILING DATE: 28-APR-1995
(J) PUBLICATION DATE: 31-OCT-1996

(xi) SEQUENCE DESCRIPTION: SEQ ID NO: 5:

Cys Thr Thr His His Val Ser Pro Asp Ala Asp Leu Ile Glu Ala Asn
1               5                   10                  15

Leu Leu Trp Arg
            20

(2) INFORMATION FOR SEQ ID NO: 6:

(i) SEQUENCE CHARACTERISTICS:
(A) LENGTH: 20 amino acids
(B) TYPE: amino acid
(C) STRANDEDNESS:
(D) TOPOLOGY: linear

(ii) MOLECULE TYPE: peptide

(iii) HYPOTHETICAL: NO

(v) FRAGMENT TYPE: internal

(vi) ORIGINAL SOURCE:
(A) ORGANISM: Hepatitis virus

(ix) FEATURE:
(A) NAME/KEY: Peptide
(B) LOCATION:6..20

(x) PUBLICATION INFORMATION:

(H) DOCUMENT NUMBER: WO 96/34013 A1
(I) FILING DATE: 28-APR-1995
(J) PUBLICATION DATE: 31-OCT-1996

(xi) SEQUENCE DESCRIPTION: SEQ ID NO: 6:

Cys Thr Thr His Gly Lys Ala Tyr Asp Val Asp Met Val Asp Ala Asn
1               5                   10                  15

Leu Phe Met Gly
            20

## Description

### Technical Field

The present invention relates to an antigenic peptide compound and an immunoassay which are useful for measuring an antibody relating to HCV (hepatitis C virus).

### Background Art

Various kinds of viruses exist on the earth, and some of them are pathogens. For example, it is known that a hepatitis virus as a cause of viral hepatitis is transmissible at the time of blood transfusion, injection, childbirth, etc.

The blood transfusion is one of the important measures for maintaining the life of a human being. However, there have heretofore been reported a large number of cases such that the blood transfusion causes viral hepatitis. In general, the viral hepatitis is classified into hepatitis A, hepatitis B and non-A non-B hepatitis.

Conventionally, it is known that hepatitis B virus (HBV) also causes viral hepatitis at the time of the blood transfusion. In recent years, a diagnostic reagent for detecting the presence of the HBV has been developed, and it has become possible to easily confirm the presence of the HBV, prior to the blood transfusion. Accordingly, the HBV infection of a human being due to the blood transfusion has entirely been prevented. On the other hand, it is also known the existence of another hepatitis (non-A non-B hepatitis) which is clearly viral, but is different from the hepatitis A caused by hepatitis A virus (HAV) and is also different from the hepatitis B caused by HBV, while the HAV and HBV have heretofore been considered as the causes of hepatitis. However, it has heretofore been considered to be difficult to confirm the cause (virus) of such non-A non-B hepatitis.

In 1989, Choo, Q-L. et al. (Science, 244, 359-362, 1989) and Kuo ,G. et al. (Science, 244, 362-364, 1989) proved the existence of the non-A non-B hepatitis virus. This hepatitis virus was named "HCV" (hepatitis C virus). It has heretofore been reported that the HCV is selectively transmissible to a human being and a chimpanzee.

The above-mentioned paper describes the following experiments. That is, a preparation of blood coagulation Factor VIII which was the same as the preparation which had caused the non-A non-B hepatitis when administered to a patient, was administered to a Chimpanzee-No. 1 thereby to cause non-A non-B hepatitis. Further, a preparation extracted from the liver of the above Chimpanzee-No. 1 was similarly administered to a Chimpanzee-No. 2 thereby to cause non-A non-B hepatitis, and thereafter, the blood plasma of this Chimpanzee-No. 2 was extracted. The resultant blood plasma of the Chimpanzee-No. 2 was further administered to a Chimpanzee-No. 3, whereby the occurrence of non-A non-B hepatitis was confirmed. The resultant blood plasma which had confirmedly cause the disease, was subjected to a concentration procedure for virus particles (Bradley, D. W. et al.; Gastroenterology, 88: 773-779, 1989) under the assumption that the virus to be obtained was flavivirus, whereby RNA of the virus was extracted. Then, a cDNA was synthesized on the basis of the thus obtained RNA, and a λgt11 library was prepared. With respect to the resultant λgt11 library, immuno-screening was conducted by using the blood serum of a convalescent chimpanzee or the blood serum of a chronic non-A non-B hepatitis patient, thereby to select a reactive clone. As a result, it was confirmed that only the clone named "5-1-1" was a cDNA fragment originated from HCV.

The above-mentioned procedure is also described in Japanese Patent Publication (KOKOKU) No. Hei 2-500880 (500880/1990), wherein the genetic sequence thereof is also shown. In addition, a genetic sequence of the HCV was also reported in Japan (Journal of Virology, 65(3), 1105 - 1113, 1991).

In the meeting of ACTA HEPATOLOGICA JAPONICA in June of 1990, a genetic sequence of the HCV structural region was shown by Okamoto of Jichi Medical School et al. (Proceedings of the 26th Meeting of ACTA HEPATOLOGICA JAPONICA in 1990). In the meeting of Japan Cancer Society in July of 1990, a genetic sequence of the HCV structural region was shown (Proceedings of the 49th meeting of Japan Cancer Society in 1990) similarly as in the above-mentioned publication by Okamoto et al. When these publications are compared with each other, it has been found that substantially no gene mutation is observed in the HCV core region, but a somewhat difference is recognized between these publications with respect to the gene arrangement or sequence of the HCV envelope region.

Thereafter, there has been proposed an antibody diagnosis using the core antigen of the HCV structural region in combination with an antigenic site of the HCV non-structural region (Rinsho-Byori (The Japanese Journal of Clinical Pathology), 40(12), 1245-1251, 1992). When such an antibody diagnosis is used, it has become possible to conduct an almost perfect screening for an HCV antibody-detected person.

On the other hand, in parallel with the above HCV diagnosis, a treatment for hepatitis C using interferon has been initiated. In this treatment, it has been found that the effect of the interferon treatment varies in accordance with the kind of the genetic type (genotype) of the HCV. The classification of the genotype has been proposed by Okamoto H. in KAN TAN SUI, 24, 7-14 (1992), and the genotypes are classified into Type-I (Proc. Natl. Acad. Sci. USA, 88, 2451-2455, 1991), Type-II (Journal of Virology, 65(3), 1105 - 1113, 1991), Type-III (Journal of General Virology, 72, 2697 - 2704, 1991), and Type-IV (Virology, 188, 331 - 341, 1992). In the meeting of ACTA HEPATOLOGICA JAPONICA held in June of 1993, it was reported that the interferon treatment for genotype-I and -II (interferon treatment: effective in 20 % of individuals tested) was lower than that for genotype-III and -IV (interferon treatment: effective in 80 % of individuals tested) (Proceedings of the 29th Meeting of ACTA HEPATOLOGICA JAPONICA, page 55, 1993).

In many cases, the administration of the interferon produces a strong side effect such as alopecia and fever, and the load thereof to the patient is heavy. Therefore, the determination of the HCV genotype prior to the treatment is extremely important in view of the prediction of the effect of the interferon administration. However, in the conventional method of determining the genotype, the HCV RNA is extracted from the specimen of a patient, a cDNA complementary to the resultant RNA is synthesized, the cDNA is amplified by a PCR (polymerase chain reaction) method, and a band corresponding to the thus amplified cDNA is identified by use of electrophoresis, thereby to determine the genotype. Since such a procedure includes complicated steps and usually lasts for 48 hours or more, it requires a long period of time and a high cost.

On the other hand, it has been reported that the HCV antibodies in the blood sera of patients can be classified into two types by using an antigen (including about 300 amino acids) which has been prepared by a genetic recombination technique (KAN TAN SUI, 22, 883-889, 1991). However, this classification is not necessarily sufficient in view of the correspondence thereof with the above-mentioned genotype, which is important in determining the interferon treatment.

As described above, the administration of the interferon requires a high cost, and further, the determination of the above-mentioned genotype, which is a prerequisite for the interferon administration, has a disadvantage such that the determination per se also requires a high cost. If the genotype of the HCV can be determined easily prior to the interferon administration, it becomes possible to easily predict the effect of the interferon administration so as to properly design the method of administrating the interferon and a guideline for the treatment using the same. In addition, it becomes possible to reduce the mental, physical and economic load to the patient. Accordingly, there has eagerly been desired a simple method of determining the HCV genotype.

Accordingly, an object of the present invention is to provide an antigen for measuring an HCV antibody, which enables simple determination of the HCV antibody wherein the genotype-I and genotype-II can be distinguished from the genotype-III and genotype-IV.

Another object of the present invention is to provide an antigen for measuring an HCV antibody, which enables the determination of the HCV antibody wherein the genotype-I and genotype-II can be distinguished from the genotype-III and genotype-IV; and further a cross-reaction or non-specific reaction is suppressed.

A further object of the present invention is to provide a method of simply measuring an HCV antibody, wherein the genotype-I and genotype-II can be distinguished from the genotype-III and genotype-IV.

A further object of the present invention is to provide a method of simply measuring an HCV antibody, wherein the genotype-I and genotype-II can be distinguished from the genotype-III and genotype-IV; and further a cross-reaction or non-specific reaction is suppressed.

A further object of the present invention is to provide a method of measuring an HCV antibody at a low cost, wherein the genotype-I and genotype-II can be distinguished from the genotype-III and genotype-IV.

### Disclosure of Invention

As a result of earnest study, the present inventor has found that the determination of an HCV antibody type (serotype) using a specific amino acid sequence based on the an HCV antigenic site (a sequence comprising at least six amino acids) not only enables simple discrimination of the genotype-I and -II of HCV from the genotype-III and -IV thereof, but also effectively suppress a cross reaction or non-specific reaction which can occur along with an antigen-antibody reaction, whereby such determination is extremely effective in achieving the above-mentioned objects.

The antigenic peptide compound according to the present invention is based on the above discovery, and more specifically, it comprises: a sequence which comprises at least six sequential amino acids and is included in an amino acid sequence represented by the following formula (1):
Arg-Lys-Thr-Ser-Glu-Arg-Ser-Gln-Pro-Arg-Gly-Arg-Arg-Gln-Pro-Ile-Pro-Lys-Ala-Arg-Arg-Pro-Glu-Gly-Arg-Thr-Trp-Ala-Gln-Pro ······ (1)

The present invention also provides an antigenic peptide compound, comprising a sequence which comprises at least six sequential amino acids and is included in an amino acid sequence represented by the following formula (2):
Arg-Lys-Thr-Ser-Glu-Arg-Ser-Gln-Pro-Arg-Gly-Arg-Arg-Gln-Pro-Ile-Pro-Lys-Asp-Arg-Arg-Ser-Thr-Gly-Lys-Ser-Trp-Gly-Lys-Pro ······ (2)

The present invention further provides an immunological method of measuring an HCV antibody, comprising: fixing any of the antigenic peptides represented by the above formula (1) and/or (2) to a carrier; binding an HCV antibody in a specimen to the peptide by utilizing an antigen-antibody reaction; and binding a labeled ligand to the HCV antibody by utilizing an antigen-antibody reaction, thereby to measure the HCV antibody in the specimen.

Herein, "ligand" refers to a substance (such as antibody and/or antigen) having a specific binding affinity to an antigen and/or antibody.

In the above-mentioned conventional determination of the genotype, the type of HCV is determined by utilizing the sequence of the HCV gene per se. On the other hand, in the present invention, the type of HCV is determined by utilizing the type of an antibody (serotype) corresponding to an antigenic site peculiar to the HCV. Such determination of the antibody type according to the present invention may be conducted within a much shorter period of time as compared with the period of time required for the conventional genotype determination (extraction of HCV RNA → synthesis of cDNA → amplification of the cDNA by PCR method → identification of the amplified band using electrophoresis). In addition, the operation steps constituting the antibody type determination according to the present invention are simple. As a result, according to the present invention, there may be provided a method of simply determining the HCV type (genotype) at a low cost.

In addition, in the present invention, the cross reaction or non-specific reaction which can occur along with an antigen-antibody reaction is effectively suppressed, as compared with that in the case of the conventional method of determining the serum type by utilizing an antigen comprising about 300 amino acids.

When a method of using an "amino acid sequence comprising 20 amino acids" as an antigen peptide (Japanese Patent Application Nos. Hei 5-272864 (i.e., No. 272864/1993) and Hei 6-207695 (i.e., No. 207695/1994); and International Patent Application No. PCT/JP94/01823), it is also possible to differentiate the HCV genotype (type-I and -II/ type-III and -IV). However, when there is used a method according to the present invention using an amino acid sequence comprising a sequence contained in an amino acid sequence comprising 30 amino acids and having at least six sequential amino acids, as compared with in a case using the above 20-amino acid sequence, the resultant specificity is not decreased, but a further improvement in the reactivity and type-determining efficiency is rather provided, although the amino-acid homology is increased.

Further, since the sensitivity to the core region is increased according to the present invention, for example, from an epidemiological viewpoint, it is possible to distinguish the past infection of HCV (e.g., based on the determination of the core region as a virus main body) and the current infection thereof (e.g., based on the determination of NS4 and/or NS5 region appearing at the time of the virus propagation) by using the serotype.

More specifically, the peptide appearing at the time of the virus propagation such as NS4 and NS5 regions immediately disappears after the cure of the hepatitis, but on the other hand, the core region can be detected even within a predetermined period of time after the cure of the hepatitis (e.g., about one to two years after the cure). Therefore, according to the present invention, it is also possible to distinguish the past HCV infection on the basis of the detection of the core region.

### Brief Description of Drawings

Figs. 1A to 1C are graphs showing the results which have been obtained by calculating the "Hydrophilicity Value", "Hydropathy Value" and "Antigenicity Value" in Example 1 with respect to the core region of HCV appearing hereinafter.

Fig. 2 is a chromatogram showing the results of HPLC analysis of an antigenic peptide compound (1) (ckk-c12) obtained in Example 2.

Fig. 3 is a chromatogram showing the results of HPLC analysis of an antigenic peptide compound (2) (ckk-c12) obtained in Example 2.

Fig. 4 is a view schematically showing the relationships among the HCV antigenic regions and the respective antigenic peptides.

Fig. 5 (Table 1) is a table showing the results of HCV serotype determination which have been obtained in Examples appearing hereinafter.

### Best Mode for Carrying Out the Invention

Hereinbelow, the present invention will be described in detail with reference to the accompanying drawings as desired.

In the description of the amino acid sequences in the present specification, the respective amino acids constituting an amino acid sequence are described in a direction of from the N-terminal amino acid (left end) toward the C-terminal amino acid (right end). For example, in the following amino acid sequence (1), "Arg" is the N-terminal, and "Pro" is the C-terminal. In the present specification, the following amino acid descriptions are used.

### (One-character description and three-character description of amino acids)

| 〈Abbrev.〉 | 〈Name〉 | 〈Abbrev.〉 | 〈Name〉 |
|---|---|---|---|
| Asp D | aspartic acid | Val V | valine |
| Asn N | asparagine | Met M | methionine |
| Thr T | threonine | Ile I | isoleucine |
| Ser S | serine | Leu L | leucine |
| Glu E | glutamic acid | Tyr Y | tyrosine |
| Gln Q | glutamine | Phe F | phenylalanine |
| Pro P | proline | Trp W | tryptophan |
| Gly G | glycine | Lys K | lysine |
| Ala A | alanine | His H | histidine |
| Cys C | cysteine | Arg R | arginine |

### (Antigenic peptide compound)

The antigenic peptide compound according to the present invention is a peptide compound which has a sequence included in the following formula (1) or (2) having thirty (30) amino acids, and includes a sequence of at least 6 (six) sequential amino acids.
Arg-Lys-Thr-Ser-Glu-Arg-Ser-Gln-Pro-Arg-Gly-Arg-Arg-Gln-Pro-Ile-Pro-Lys-Ala-Arg-Arg-Pro-Glu-Gly-Arg-Thr-Trp-Ala-Gln-Pro ······· (1) (ckk-c12; core-1, corresponding to core region)

### (one-character description: RKTSE RSQPR GRRQP IPKAR RPEGR TWAQP)

Arg-Lys-Thr-Ser-Glu-Arg-Ser-Gln-Pro-Arg-Gly-Arg-Arg-Gln-Pro-Ile-Pro-Lys-Asp-Arg-Arg-Ser-Thr-Gly-Lys-Ser-Trp-Gly-Lys-Pro ······· (2) (ckk-c22; core-2, corresponding to core region)

### (one-character description: RKTSE RSQPR GRRQP IPKDR RSTGK SWGKP)

According to the present inventor's discovery, any of sequences which have various lengths, constitute the sequence represented by the above formula (1) or (2), and include at least sequential six amino acids may preferably be used as an antigen, similarly as the peptide (1) and/or (2). The number of the amino acids constituting each of the "amino acid sequences having various lengths" may preferably be 21 or larger (more preferably 25 or larger), in view of the balance between the ability to discriminate HCV, and the suppression of the cross-reaction/non-specific reaction.

Specific examples of such amino acid sequences having various lengths (including amino acids of not less than 6 and not more than 30) may include those as shown below.

Amino acid sequences included in the above peptide compound (1)
Arg-Ser-Gln-Pro-Arg-Gly-Arg-Arg-Glu-Pro-Ile-Pro-Lys-Ala-Arg-Arg-Pro-Glu-Gly-Arg-Thr-Trp-Ala-Gln-Pro

### (One-character description: RSQPR GRRQP IPKAR RPEGR TWAQP)

Amino acid sequences included in the above peptide compound (2)
Arg-Ser-Glu-Pro-Arg-Gly-Arg-Arg-Gln-Pro-Ile-Pro-Lys-Asp-Arg-Arg-Ser-Thr-Gly-Lys-Ser-Trp-Gly- Lys-Pro

### (One-character description: RSQPR GRRQP IPKDR RSTGK SWGKP)

According to the present inventor's discovery, any of peptides (including 31 or more amino acids) which comprise the sequence represented by the above formula (1) or (2) may preferably be used as an antigen, similarly as the peptide (1) or (2). The number of the amino acids constituting such a peptide may preferably be 40 or less when the peptide is chemically synthesized; and may preferably be 100 or less when the peptide is synthesized by using a recombinant technique.

The method of obtaining the above-mentioned antigenic peptides (peptide compounds including at least six amino acids) is not particularly limited. The peptide may be synthesized by using a chemical method, or may be produced by using a genetic engineering technique (for example, genetic recombinant technique, i.e., recombinant method). When a chemical technique is used, it is preferred to link the respective amino acids by use of a solid-phase technique, in view of easiness in the purification of an intermediate product, etc. It is further preferred to synthesize the peptide by means of an automatic peptide synthesizing apparatus (e.g., "430 A" Peptide Synthesizer mfd. by Applied Bio-Systems Co.).

### (Confirmation of antigenicity of antigenic peptide)

The sequences of the above peptides (1) and (2) have been discovered on the basis of the "extraction" of an antigenic site from the amino acid sequence of the core region of HCV (as described in Example 1 appearing hereinafter). Further, the present inventor has investigated the HCV genotype by use of an enzyme immunoassay technique (ELISA or EIA) using these peptides (1) and (2), and has confirmed the specificity of such a technique by using specimens of patients, the HCV genotypes of which have separately been identified (as described in Examples 4 and 5 appearing hereinafter).

### (Other antigenic peptides)

By use of the above-mentioned peptide (1) and (2) (both of which are sequences corresponding to the core region of HCV), it is possible to sufficiently determine the serotype, as descried hereinafter. However, in view of a further increase in the accuracy in the serotype determination, or in view of an expansion in the applicable scope of the serotype determination, it is preferred to use the above-mentioned peptide (1) and/or (2) in combination with another antigenic peptide. As such "other antigenic peptide", the following peptides (3) to (6) are usable.
Ile-Ile-Leu-Ser-Gly-Arg-Pro-Ala-Ile-Val-Pro-Asp-Arg-Glu-Leu-Leu-Tyr-Gln-Glu-Phe-Asp-Glu-Met-Glu-Glu-Cys-Ala-Ser-His-Leu-Pro-Tyr-Ile-Glu-Gln-Gly-Met-Gln-Leu-Ala ······· (3)
(ckk-n1(40); corresponding to NS-4 region)

### (One-character description: IILSG RPAIV PDREL LYQEF DEMEE CASHL PYIEQ GMQLA)

Leu-His-Val-Asn-Gln-Arg-Ala-Val-Val-Ala-Pro-Asp-Lys-Glu-Val-Leu-Tyr-Glu-Ala-Phe-Asp-Glu-Met-Glu-Glu-Cys-Ala-Ser-Arg-Ala-Ala-Leu-Ile-Glu-Glu-Gly-Gln-Arg-Ile-Ala ·······(4)
(ckk-n2(40); corresponding to NS-4 region)

### (One-character description: LHVNQ RAVVA PDKEV LYEAF DEMEE CASRA ALIEE GQRIA)

Cys-Thr-Thr-His-His-Val-Ser-Pro-Asp-Ala-Asp-Leu-Il e-Glu-Ala-Asn-Leu-Leu-Trp-Arg ·······(5)
(ckk-n3; corresponding to NS-5 region)

### (One-character description: CTTHH VSPDA DLIEA NLLWR)

Cys-Thr-Thr-His-Gly-Lys-Ala-Tyr-Asp-Val-Asp-Met-Va l-Asp-Ala-Asn-Leu-Phe-Met-Gly ·······(6)
(ckk-n4; corresponding to NS-5 region)

### (One-character description: CTTHG KAYDV DMVDA NLFMG)

With respect to the above peptides (3) to (6), it is possible to confirm the antigenicity thereof, to prepare the peptides, etc., in the same manner as in the case of the peptides (1) and (2) corresponding to the core region.

According to the present inventor's discovery, any of sequences which have various lengths, constitute the sequence represented by the above formulas (3) to (6) comprising 40 or 20 amino acids, and includes at least six sequential amino acids may preferably be used as an antigen, similarly as each of the peptides (3), (4), (5), and/or (6).

Specific examples of such amino acid sequences having various lengths (including amino acids of not less than 6 and not more than 30) may include those as shown below.

Amino acid sequences included in the above peptide compound (3)
Ile-Ile-leu-Ser-Gly-Arg-Pro-Ala-Ile-Val-Pro-Arg-Arg-Glu-Leu-Leu-Tyr-Gln-Glu-Phe-Asp-Glu-Met-Glu-Glu-Cys-Ala-Ser-His-Leu ·······(7)

### (One-character description: IILSG RPAIV PDREL LYQEF DEMEE CASHL)

Amino acid sequences included in the above peptide compound (4)
Leu-His-Val-Asn-Gln-Arg-Ala-Val-Val-Ala-Pro-Arg-Lys-Glu-Val-Leu-Tyr-Glu-Ala-Phe-Asp-Glu-Met-Glu-Glu-Cys-Ala-Ser-Arg-Ala········(8)

### (One-character description: LHVNQ RAVVA PDKEV LYEAF DEMEE CASRA)

Amino acid sequences included in the above peptide compound (5)
Val-Ser-Pro-Asp-Ala-Asp-Leu-Ile-Glu-Ala-Asn-Leu-Leu-Trp-Arg ········(9)

### (One-character description: VSPDA DLIEA NLLWR)

Amino acid sequences included in the above peptide compound (6)
Lys-Ala-Tyr-Asp-Val-Asp-Met-Val-Asp-Ala-Asn-Leu-Phe-Met-Gly ········ (10)

### (One-character description: KAYDV DMVDA NLFMG)

According to the present inventor's discovery, any of peptides (including amino acids of 16 - 31 or more) which comprise any of the sequences represented by the above formulas (3) to (6) may preferably be used as an antigen, similarly as each of the peptides (3) to (6). The number of amino acids constituting such a peptide may preferably be 40 or less when the peptide is chemically synthesized; and may preferably be 100 or less when the peptide is synthesized by using a recombinant technique.

According to the present inventor's discovery, when any of the above peptides (1) to (6) is used singly as an antigen, in view of the accuracy in the serotype determination, the order of preference in the use thereof as an antigen is as follows. In the description in this portion, the peptides (1) and/or (2) are inclusively referred to as "core"; the peptides (3) and/or (4) are inclusively referred to as "NS-4"; and the peptides (5) and/or (6) are inclusively referred to as "NS-5".

Preference order: core > NS-4 > NS-5

On the other hand, when at least two kinds of the above peptides core, NS-4, and NS-5 are used in combination, the order of preference in the use thereof as an antigen is as follows, in view of the accuracy in the serotype determination.

Preference order: (core) + (NS-4) > (core) + (NS-5)

Further, when at least three kinds of the above peptides core, NS-4, and NS-5 are used in combination, the order of preference in the use thereof as an antigen is as follows, in view of the accuracy in the serotype determination.

Preference order: (core) + (NS-4) + (NS-5) > (core) + (NS-4) + (NS-5)

### (Method of measuring HCV antibody)

By utilizing the specific affinity of the above antigenic peptide to an HCV antibody, the HCV antibody in a sample or specimen may be measured immunologically. The immunological measuring method to be used in the present invention is not particularly limited, but it is possible to utilize any of known immunoassay methods without particular limitation. Specific examples of such immunoassay methods may include: enzyme immunoassay, radio immunoassay (RIA) fluorescence immunoassay (FIA), etc. In these immunoassay techniques, any of known procedures (such as competitive method, double-antibody method, and sandwich method) may be used without particular limitation. With respect to these known measuring techniques, corresponding papers (e.g., "Enzyme Immunoassay" (3rd edition) written by Eiji Ishikawa, pages 180 et seq., published by Igaku Shoin, with respect to EIA) may be referred to.

According to the present invention, an HCV antibody may be immunologically measured by use of the above-mentioned antigenic peptide, whereby the kind (serotype) of the HCV antibody contained in a specimen may be determined (For example, as described hereinbelow, it is possible to determine whether the serotype of the HCV antibody is classified into group-I or group-II). As described hereinbelow, the group-I of HCV serotype corresponds to the HCV genotype-I and -II; and the group-II of HCV serotype corresponds to the HCV genotype-III and -IV. Accordingly, it is possible to easily determine the HCV genotype.

In the above immunoassay, as desired, it is possible that the antigenic peptide is attached or solid phase-fixed to a carrier (or support), and the kind of the antibody against the HCV antigen (i.e., serotype of HCV) is determined by utilizing the specific affinity of the antigenic peptide to the HCV antibody. Specific examples of the carrier or support to be used for such a purpose may include: bovine serum albumin (BSA), a polypeptide preferably having a molecular weight of about 5×10⁴ to 10×10^{4,} wells of a micro-plate, a polystyrene ball (or polystyrene bead) preferably having a diameter of about 0.1 µm to 6 mm, etc.

As described hereinabove, the above peptides (1) to (2) may suitably be used for the measurement of an HCV antibody, and may also be used for an antigen or immunogen for producing a vaccine for HCV. For example, such a vaccine may be prepared by using a genetic engineering technique (recombinant technique).

Hereinbelow, the present invention will be described in more detail with reference to Examples.

### Example 1

### (Identification of antigenic site by use of Hydrophilicity Value)

First, the amino acid sequences of a non-structural region and a structural region of HCV were determined, on the basis of the sequence of the antigenic peptide synthesized in Geoffrey RS; Nature, 302, 490-495 (1983) and the genetic sequence of HCV (Proceedings of 26th Meeting of ACTA HEPATOLOGICA JAPONICA, 1990; Proceedings of 49th Meeting of Japan Cancer Society, 1990; Proc. Natl. Acad. Sci. USA, 88, 2451-2455, 1991; Journal of Virology, 65(3), 1105 - 1113, 1991; Journal of General Virology, 72, 2697-2704, 1991; and Virology, 188, 331 - 341, 1992).

On the basis of the thus determined amino acid sequence, the antigenic site thereof was evaluated by using a method described in Hopp TP; Proc. Natl. Acad. Sci. 78, 3824 - 3828 (1981). More specifically, the evaluation was conducted in the following manner by using the above "Hydrophilicity Value" of amino acids.

Based on the thus determined amino acid sequence constituting the HCV, the Hydrophilicity Values of respective sequential six amino acids were summed up. Such calculations were conducted with respect to all the regions of the amino acid sequences of HCV, thereby to calculate the strength of their hydrophilic properties and hydrophobic properties. The region showing a high hydrophilic property was treated as a site showing an antigenicity.

At this time, it was also possible to calculate the Hydropathy Value as described in Jack K., Mol. Biol. 157, 105 (1982); and the Antigenicity Value as described in Alan MS., Science, 227, 429 (1985) in a similar same manner as in the calculation of the above Hydrophilicity Value.

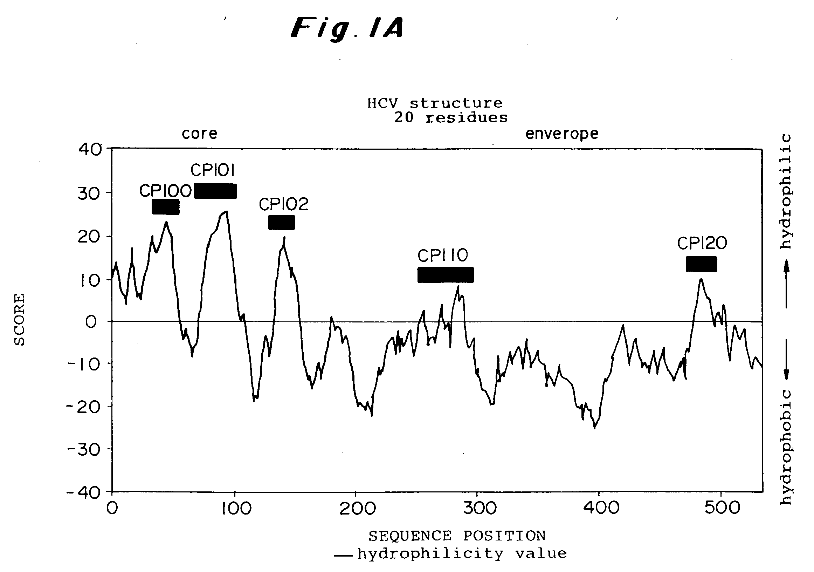

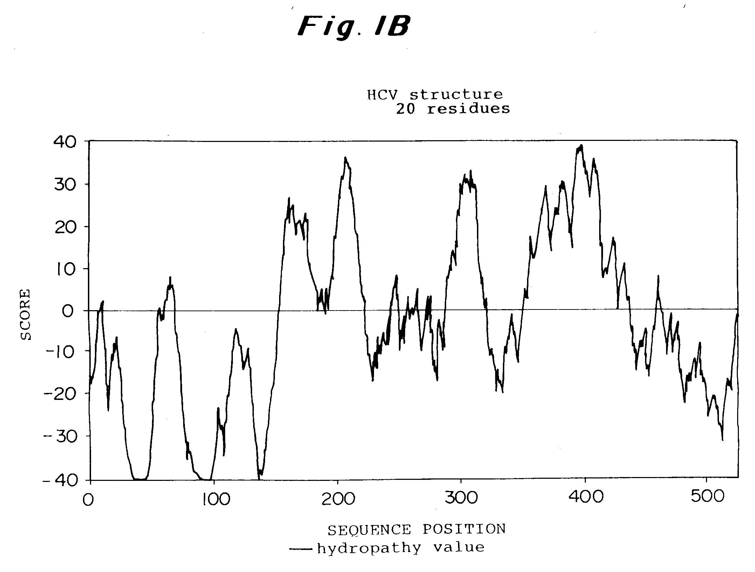

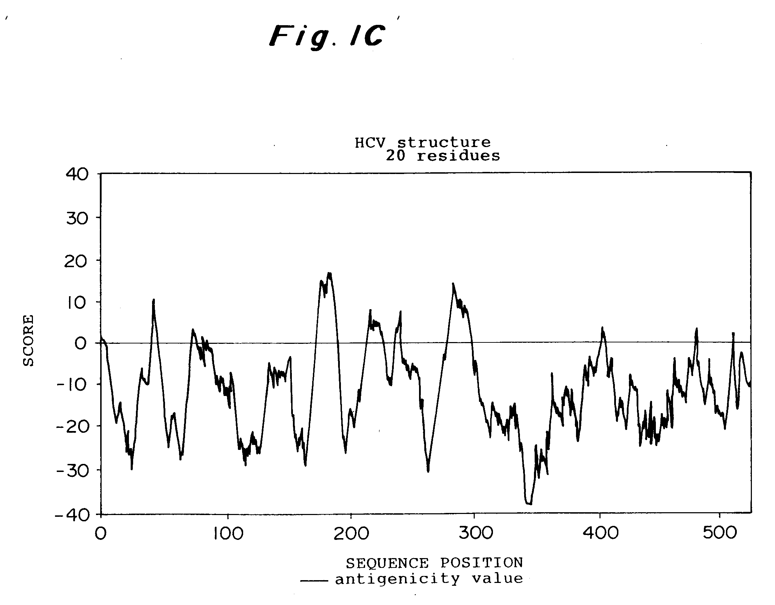

The results of the calculation using the above Hydrophilicity Value are shown in graphs of Fig. 1 (core region). This graph also shows the results of the calculation using the above Hydropathy Value and Antigenicity Value, in combination.

Based on the results obtained by using Fig. 1, regions capable of being an antigen of hepatitis C were extracted. Further, among the thus determined regions, regions having a common amino acid sequence with respect to the genotype-I and -II, and regions having a common amino acid sequence with respect to the genotype-III and -IV were determined. As a result of such evaluation, there were discovered the following ckk-c12 and ckk-c22 as antigenic amino acid sequences for the core region satisfying such conditions.
Arg-Lys-Thr-Ser-Glu-Arg-Ser-Gln-Pro-Arg-Gly-Arg-Arg-Gln-Pro-Ile-Pro-Lys-Ala-Arg-Arg-Pro-Glu-Gly-Arg-Thr-Trp-Ala-Gln-Pro ·······(ckk-c12)
Arg-Lys-Thr-Ser-Glu-Arg-Ser-Gln-Pro-Arg-Gly-Arg-Arg-Gln-Pro-Ile-Pro-Lys-Asp-Arg-Arg-Ser-Thr-Gly-Lys-Ser-Trp-Gly-Lys-Pro ·······(ckk-c22)

### Example 2

### (Preparation of antigen of hepatitis C)

The above-mentioned HCV antigenic peptides (ckk-c12) and (ckk-c22) were synthesized in the following manner.

The peptides were synthesized by means of an automatic peptide synthesizing apparatus of "430A" Peptide Synthesizer mfd. by Applied Biosystems, by use of symmetric anhydrides of t-Boc amino acids as reagents. The resultant synthesized peptide was dissolved in anisole dimethyl sulfide para-thio cresol, and thereafter, was subjected to a reaction at 0 - 5 °C for one hour in the presence of hydrofluoric acid, thereby to remove the protecting group (with reference to S. Sakakibara, Bull. Chem. Soc. Jpn., 40, 2164, (1967)).

The resultant crude crystals obtained by the removal of the protecting group was dissolved in 2N-acetic acid, and then extracted by use of ether, and the resultant extract was freeze-dried. The freeze-dried product was purified by means of an HPLC (high-performance liquid chromatography).

This HPLC purification was conducted by using a column (SISEIDO Capsule-Pack C-18 SG120, diameter: 46 mm, length: 250 mm) in a manner such that a gradient was applied to the mobile phase at a flow rate of 12 ml/min by use of a solution comprising 0.1 % of trifluoroacetic acid (TFA) and 5 % of acetonitrile (CH₃CN) in pure water; and a solution comprising 0.1 % of TFA and 50 % of acetonitrile (CH₃CN) in pure water.

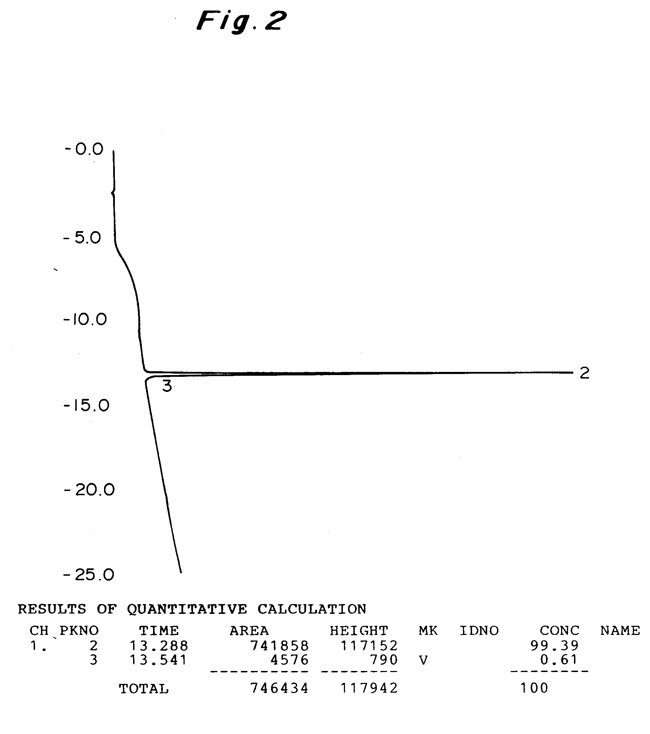

The chromatogram obtained at this time is shown in Fig. 2 (ckk-c12).

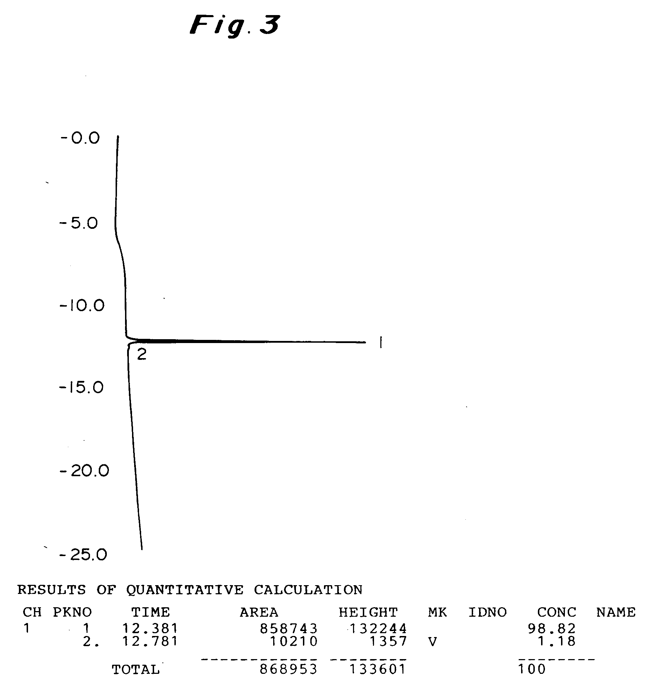

In the same manner as described above, another synthesized antigenic peptide ckk-c22 was purified by use of HPLC. The chromatogram obtained at this time is shown in Fig. 3 (ckk-c22).

In the same manner as described above, the above-mentioned six kinds of synthesized antigenic peptides ckk-n1(40) and ckk-n2(40) (peptides corresponding to the NS-4 region), ckk-n3 and ckk-n4 (peptides corresponding to the NS-5 region), and ckk-c1 and ckk-c2 (20-amino acid peptides corresponding to the core region) were synthesized.
Ile-Ile-Leu-Ser-Gly-Arg-Pro-Ala-Ile-Val-Pro-Asp-Arg-Glu-Leu-Leu-Tyr-Gln-Glu-Phe-Asp-Glu-Met- Glu-Glu-Cys-Ala-Ser-His-Leu-Pro-Tyr-Ile-Glu-Gln-Gly-Met-Gln-Leu-Ala ·······(3) (ckk-n1(40), corresponding to the NS-4 region)

### (One-character description: IILSG RPAIV PDREL LYQEF DEMEE CASHL PYIEQ GMQLA)

Leu-His-Val-Asn-Gln-Arg-Ala-Val-Val-Ala-Pro-Asp-Lys-Glu-Val-Leu-Tyr-Glu-Ala-Phe-Asp-Glu-Met-Glu-Glu-Cys-Ala-Ser-Arg-Ala-Ala-Leu-Ile-Glu-Glu-Gly-Gln-Arg-Ile-Ala ·······(4) (ckk-n2(40), corresponding to the NS-4 region)

### (One-character description: LHVNQ RAVVA PDKEV LYEAF DEMEE CASRA ALIEE GQRIA)

Cys-Thr-Thr-His-His-Val-Ser-Pro-Asp-Ala-Asp-Leu-Ile-Glu-Ala-Asn-Leu-Leu-Trp-Arg ······· (5) (ckk-n3, corresponding to the NS-5 region)

### (One-character description: CTTHH VSPDA DLIEA NLLWR)

Cys-Thr-Thr-His-Gly-Lys-Ala-Tyr-Asp-Val-Asp-Met-Val-Asp-Ala-Asn-Leu-Phe-Met-Gly ······· (6) (ckk-n4, corresponding to the NS-5 region)

### (One-character description: CTTHG KAYDV DMYDA NLFMG)

Gly-Arg-Arg-Gln-Pro-Ile-Pro-Lys-Ala-Arg-Arg-Pro-Glu-Gly-Arg-Thr-Trp-Ala-Gln-Pro ······· (7) (ckk-c1, corresponding to the core region)

### (One-character description: GRRQP IPKAR RPEGR TWAQP)

Gly-Arg-Arg-Gln-Pro-Ile-Pro-Lys-Asp-Arg-Arg-Ser-Thr-Gly-Lys-Ser-Trp-Gly-Lys-Pro ·······(8) (ckk-c2, corresponding to the core region)

### (One-character description: GRRQP IPKDR RSTGK SWGKP)

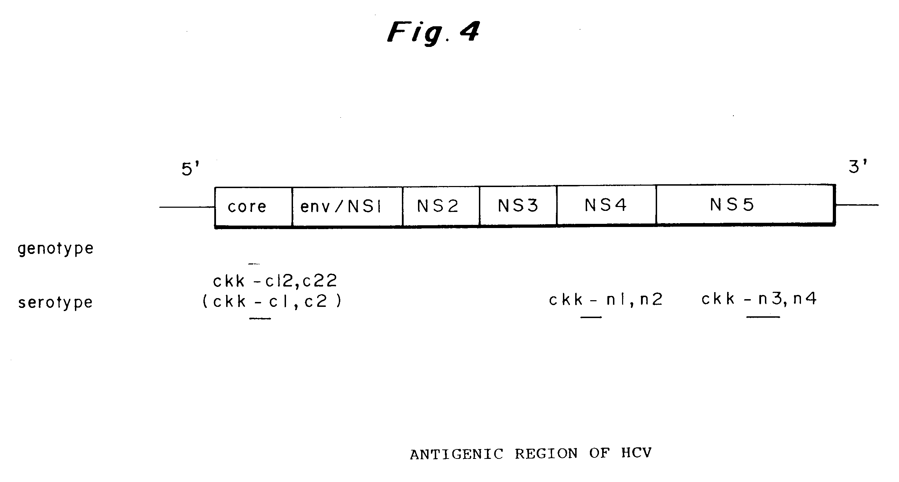

The relationships among the above-mentioned antigenic peptides and the antigenic regions of the HCV are schematically shown in Fig. 4.

### Example 3

### (Preparation of plate for ELISA)

The antigenic peptide ckk-c12 (corresponding to the core region) obtained in Example 2 was dissolved in 0.15M NaCl - 0.10M Na₂HPO₄ - NaH₂PO₄ · 2H₂O buffer (PBS), pH = 7.0 so as to provide a concentration of 0.04 g/ml. The resultant peptide solution was poured into wells of a 96-well micro-plate (trade name: ELISA PLATE 68667, mfd. by NUNC Co.) so that 100 µl of the solution was poured into each of the wells. Then, the micro-plate was left standing at 37 °C for 60 minutes, thereby to solid phase-fix the above antigenic peptide to the micro-plate.

An excess amount of the above peptide solution was removed, and thereafter, the micro-plate was washed three times with 0.01M PBS (pH = 7.0). After the washing solution was removed, a solution of gelatine in 0.01M PBS (pH = 7.0) having a concentration of 0.1 % was poured into the wells so that 300 µl of the solution was poured into each of the wells, and then the micro-plate was left standing at 37 °C for 60 minutes, thereby to coat the micro-plate with the gelatine. After an excess of the above gelatine solution was removed, the micro-plate was washed three times with 0.01M PBS (pH = 7.0) containing Tween-20 at a concentration of 0.05 %.

The resultant plate coated with the gelatine was dried at 25 °C for six hours, and thereafter, was stored at 4 °C until the plate was used for analyzing specimens.

### Example 4

### (Analysis of specimens)

Specimens (20 kinds) were preliminarily subjected to the HCV genotype determination using a PCR method as described in the above-mentioned paper (Okamoto H., KAN TAN SUI, 24, 7 - 14 (1992)) in Hachioji Laboratory of SRL, Inc., thereby to determine the HCV genotypes thereof. In combination therewith, there were also measured the amounts of the HCV-RNA (unit: mEQ/ml) of each specimen, the amounts of the HCV-RNA of which had been measured by using a commercially available HCV-RNA measuring reagent (trade name: Quantiplex HCV-RNA, mfd. by Chiron Co.). (A small amount of the HCV-RNA shows a small number of HCV virus particles.)

By use of the above specimens, the HCV genotypes of which had been determined in this manner by using the PCR method, the specificity of the antigenic peptide prepared in Example 2 was confirmed in the following manner. This investigation was carried out according to the following procedure by using the micro-plate prepared in Example 3 to which the antigenic peptide had been fixed.

Each of the above specimens was diluted with 0.01M PBS (pH = 7.0) containing 0.01 % of BSA and 0.05 % of Tween-20 so that the resultant volume after the dilution was 50 times the volume of the original specimen. The thus diluted specimen was poured into each of the micro-plate wells in an amount of 100 µl per one well, and then was allowed to react with the above antigenic peptide at 37 °C for 60 minutes (reaction between antigenic peptide - HCV antibody).

After the reaction, the micro-plate was washed five times with 0.01M PBS (pH = 7.0) containing 0.05 % of Tween-20, and the washing liquid was removed. Thereafter, a peroxidase-labeled anti-human IgG antibody having a concentration of 0.1 µg/20ml (mfd. by KPL Co.) was added to each of the wells in an amount of 100 µl per one well, and was allowed to cause a reaction at 37 °C for 60 minutes (reaction between HCV antibody - POD-labeled anti-human IgG antibody).

After the reaction, the micro-plate was washed five times with 0.01M PBS (pH = 7.0) containing 0.05 % of Tween-20, and the washing liquid was removed. Thereafter, 100 µl of 0.023 % of hydrogen peroxide, and 100 µl of 0.1 M citric acid - Na2HPO4 buffer containing 0.005 % of o-phenylene diamine (OPD) were poured into each of the wells, and was allowed to cause a reaction at 37 °C for 30 minutes (reaction for measuring the activity of peroxidase). After the reaction, 50 µl of 5N-sulfuric acid wad added to each of the wells to stop the reaction, the absorbance (ABS or OD) at a wavelength of 491 nm was measured by means of a spectrophotometer (trade name: Plate Reader NJ-2001, mfd. by Nihon Inter-Med Co.).

### Example 5

### (Measurement of specimen using antigenic peptide ckk-c22)

A micro-plate to which an antigenic peptide ckk-c22 had been solid-phase fixed was prepared in the same manner as in Example 3 except for using the antigenic peptide ckk-c22 obtained in Example 2 instead of the antigenic peptide ckk-c12 used in Example 3. Specimens (20 kinds), the HCV genotypes of which had preliminarily been determined, were analyzed in the same manner as in Example 4 except for using the thus obtained micro-plate.

Then, the serotypes of these specimens were determined on the basis of the thus obtained measured absorbance data and those obtained in the above Example 4. In this serotype determination, the absorbance obtained by using the antigenic peptide ckk-c12 (i.e., a peptide corresponding to serotype-1) and the absorbance obtained by using the antigenic peptide ckk-c22 (i.e., a peptide corresponding to serotype-2) were compared with each other. When one of the above absorbance values was 1.5 times or more the other of the above absorbance values, the serotype was judged to be a serotype corresponding to the higher absorbance value (i.e., when the higher one is ckk-c12 side, it was judged to be serotype-1; and when the higher one is ckk-c22 side, it was judged to be serotype-2).

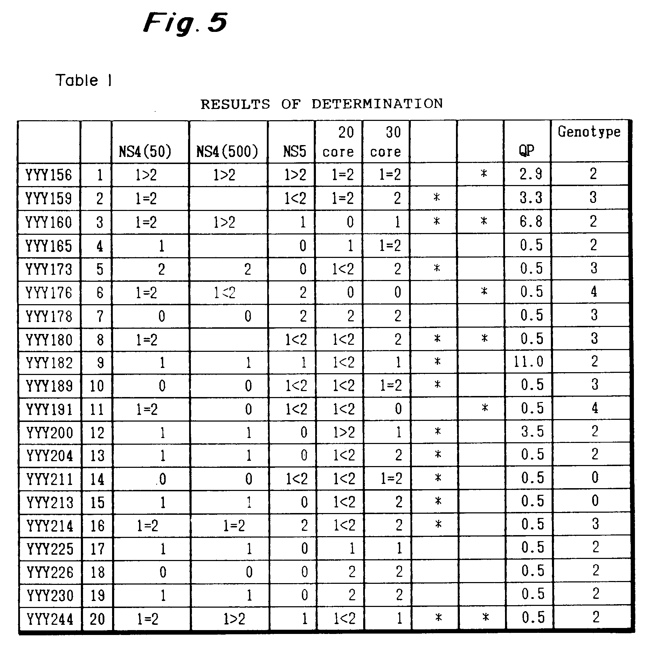

The results obtained in the above serotype determination are shown in the seventh column (represented by "30 core") of Fig. 5 (Table 1).

In the above Fig. 5 (Table 1), the respective data have the following meanings.
First column: identification symbols of the specimens;
Second column: identification numbers of the specimens; and
Seventh column: results of the serotype determination obtained by using the ckk-c12 and ckk-c22 comprising 30 amino acids and corresponding to the core region.

In the above results of the serotype determination shown in Fig. 5 (N = 20), the symbol "1" denotes "type-1", the symbol "2" denotes "type-2", the symbol "0" denotes "negative" (determination was impossible), and the symbol "1=2" denotes "mixed type" (i.e., "1+2"; that is, a case wherein either one of the absorbance value is below 1.5 times the other absorbance value). The symbol "1<2" denotes a case wherein both of the measured values were below the cut-off value (OD value = 0.5), but a relationship of (1<2) was obtained in terms of the comparison of these OD values themselves. On the other hand, the symbol "1>2" denotes a case wherein both of the measured values were below the cut-off value (OD value = 0.5), but a relationship of (1>2) was obtained in terms of the comparison of these OD values themselves.

The results obtained in the above procedure were summarized, and were compared with the results of the genotype determination (as shown in the ninth column in Fig. 5 (Table 1) denoted by "genotype"). As a result, the following relationships were obtained (with reference to Hepatology, 16(4) ,886, 1992).

### 〈Relationships between serotype and genotype〉

| Genotype determination (PCR) | serotype determination (core antigen) |
|---|---|
| I | Group I |
| II | Group I |
| III | Group II |
| IV | Group II |
| V | - |

(In the above table, "genotype V" also includes such cases, the genotype determinations of which were impossible, or indistinguishable (inclusive of "mixed types") by use of the PCR method.)

### Example 6

### (Measurement of specimens using antigenic peptides ckk-n1 and ckk-n2)

A micro-plate to which an antigenic peptide had been solid-phase fixed was prepared in the same manner as in Example 3 except for using the antigenic peptide ckk-n1 or ckk-n2 corresponding to the NS-4 region obtained in Example 2, respectively, instead of the antigenic peptide ckk-c12 used in Example 3.

Specimens (20 kinds), the HCV genotypes of which had preliminarily been determined, were analyzed in the same manner as in Examples 4 and 5 except for using the thus obtained micro-plate.

In the serotype determination obtained by the method of this Example, in the same manner as in the above Example 5, the absorbance (ckk-n1) and the absorbance (ckk-n2) were compared with each other. When one of the above absorbance values was 1.5 times or more the other of the above absorbance values, the serotype was judged to be a serotype corresponding to the higher absorbance value (i.e., when the higher one is ckk-n1 side, it was judged to be serotype-1; and when the higher one is ckk-n2 side, it was judged to be serotype-2).

The results obtained in this Example are inclusively shown in the third column "NS-4(50)" of Fig. 5 (Table 1).

The results obtained in this Example were summarized, and were compared with the results of the genotype determination. As a result, the following relationships were obtained, similarly as in Examples 4 and 5.

### 〈Relationships between serotype and genotype〉

| Genotype determination (PCR) | serotype determination (NS-4) |
|---|---|
| I | Group I |
| II | Group I |
| III | Group II |
| IV | Group II |
| V | - |

### Example 7

### (Measurement in combination with change in dilution factor (or magnification) for specimen)

For the purpose of determining the serotypes of some specimens used in Example 6 (dilution factor = 50 times), the absorbance data of which were considered to be nearly saturated, on the basis of the accurately measured absorbance data thereof, the same measurement procedure was repeated while changing the dilution factor for the specimen to be measured to 500 times.

More specifically, the measurement of the absorbance and the determination of the serotype were conducted in the same manner as in Example 6 except for changing the dilution factor for the specimen to 500 times.

The thus obtained results of the serotype determination are shown in the fourth column "NS4 (500)" of Fig. 5 (Table 1) appearing hereinafter.

### Example 8

### (Measurement of specimens using antigenic peptides ckk-n3 and ckk-n4)

A micro-plate to which an antigenic peptide had been solid-phase fixed was prepared in the same manner as in Example 3 except for using the antigenic peptide ckk-n3 or ckk-n4 corresponding to the NS-5 region obtained in Example 2, respectively, instead of the antigenic peptide ckk-c12 used in Example 3.

Specimens (20 kinds), the HCV genotypes of which had preliminarily been determined, were analyzed in the same manner as in Examples 4 and 5 except for using the thus obtained micro-plate.

In the serotype determination obtained by the method of this Example, in the same manner as in Example 5, the absorbance (ckk-n3) and the absorbance (ckk-n4) were compared with each other. When one of the above absorbance values was 1.5 times or more the other of the above absorbance values, the serotype was judged to be a serotype corresponding to the higher absorbance value (i.e., when the higher one is ckk-n3 side, it was judged to be serotype-1; and when the higher one is ckk-n4 side, it was judged to be serotype-2).

The thus obtained results of the serotype determination are shown in the fifth column "NS5 (500)" of Fig. 5 (Table 1) appearing hereinafter.

The results obtained in this Example were summarized, and were compared with the results of the genotype determination. As a result, the following relationships were obtained, similarly as in Examples 4 and 5.

### 〈Relationships between serotype and genotype〉

| Genotype determination (PCR) | serotype determination (NS-5) |
|---|---|
| I | Group I |
| II | Group I |
| III | Group II |
| IV | Group II |
| V | - |

### Example 9

### (Measurement of specimens using antigenic peptides ckk-c1 and ckk-c2)

A micro-plate to which an antigenic peptide had been solid-phase fixed was prepared in the same manner as in Example 3 except for using the antigenic peptide ckk-c1 or ckk-c2 (each of which was a peptide comprising 20 amino acids) corresponding to the core region obtained in Example 2, respectively, instead of the antigenic peptide ckk-c12 used in Example 3.

Specimens (20 kinds), the HCV genotypes of which had preliminarily been determined, were analyzed in the same manner as in Examples 4 and 5 except for using the thus obtained micro-plate.

The thus obtained results of the serotype determination are shown in the sixth column "20 core" of Fig. 5 (Table 1) appearing hereinafter.

In the serotype determination obtained by the method of this Example, in the same manner as in the above Table 1, the absorbance (ckk-c1)and the absorbance (ckk-c2) were compared with each other. When one of the above absorbance values was 1.5 times or more the other of the above absorbance values, the serotype was judged to be a serotype corresponding to the higher absorbance value (i.e., when the higher one is ckk-c1 side, it was judged to be serotype-1; and when the higher one is ckk-c2 side, it was judged to be serotype-2).

The results obtained in this Example were summarized, and were compared with the results of the genotype determination. As a result, the following relationships were obtained, similarly as in Examples 4 and 5.

### 〈Relationships between serotype and genotype〉

| Genotype determination (PCR) | serotype determination (core) |
|---|---|
| I | Group I |
| II | Group I |
| III | Group II |
| IV | Group II |
| V | - |

The results obtained in this Example were summarized, and were compared with the results of the genotype determination. As a result, the following relationships were obtained, similarly as in Examples 4 and 5.

### 〈Relationships between serotype and genotype〉

| Genotype determination (PCR) | serotype determination (core) |
|---|---|
| I | Group I |
| II | Group I |
| III | Group II |
| IV | Group II |
| V | - |

The results of the serotype determination obtained in the above Examples 4 - 9 are inclusively shown in Fig. 5 (Table 1). In the above Fig. 5 (Table 1), the data in the respective columns have the following meanings.
First column: identification symbols of the specimens;
Second column: identification numbers of the specimens; and
Third column: results of the serotype determination obtained by using the antigenic peptides ckk-n1 and ckk-n2 corresponding to the NS-4 region (wherein the dilution factor was 50 times).
Fourth column: results of the serotype determination obtained by using the antigenic peptides ckk-n1 and ckk-n2 corresponding to the NS-4 region (wherein the dilution factor was 500 times).
Fifth column: results of the serotype determination obtained by using the antigenic peptides ckk-n3 and ckk-n4 corresponding to the NS-5 region.
Sixth column: results of the serotype determination obtained by using the antigenic peptides ckk-c1 and ckk-c2 (each of which comprised 20 amino acids) corresponding to the core region.
Seventh column: results of the serotype determination obtained by using the antigenic peptides ckk-c12 and ckk-c22 (each of which comprised 30 amino acids) corresponding to the core region.
Eighth column: results of the amount measurement of the HCV-RNA (unit: mEQ/ml) of each specimen obtained by using a commercially available HCV-RNA measuring reagent (trade name: Ouantiplex HCV-RNA, mfd. by Chiron Co.). A small amount of the HCV-RNA shows a small number of HCV virus particles.

As shown in the above Fig. 5 (Table 1), the serotypes of a portion of 5/20 = 25 % could be determined, when 20-amino acid sequences (ckk-c1 and ckk-c2) were used as the core-antigen peptide (as shown in the data in Example 9, the column denoted by "20 core"). On the other hand, the serotypes of a portion of 14/20 = 70 % could be determined, when 30-amino acid sequences (ckk-c12 and ckk-c22) were used as the core-antigen peptide (as shown in the data in Example 9, the column denoted by "30 core"). Particularly, when the 30-amino acid sequences (ckk-c12 and ckk-c22) according to the present invention were used, good serotype determination could be conducted, even when only a small amount of the HCV-DNA was present as shown in the eighth column denoted by "OP".

Further, as described above, the genotype-I and genotype-II showed a good correlation with the serotype-I. On the other hand, the genotype-III and genotype-IV showed a good correlation with the serotype-II.

### Industrial Applicability

As described hereinabove, the present invention provides an antigenic peptide having an amino acid sequence peculiar to the HCV, and a method of measuring an HCV antibody by using such a peptide.

According to the present invention, it is possible to enhance the sensitivity to the core region so as to judge the serotype of a specimen simply and accurately, while suppressing a cross reaction or non-specific reaction. Therefore, according to the present invention, for example, it is possible to preliminarily predict the effect of interferon treatment on the basis of the simple and accurate serotype determination.

Further, it is possible to simply observe the course of the curing or treatment for hepatitis C, on the basis of the measurement of the HCV antibody titer according to the present invention. More specifically, according to the present invention, it is also possible to utilize the serotype from an epidemiological viewpoint, and to obtain a knowledge on the past infection (antibody titer with respect to the core region) and on the current hepatitis C (antibody titer with respect to the NS-4 and/or NS-5 region).

Further, the present invention provides an antigen for measuring an HCV antibody, which enables simple determination of the HCV antibody wherein the HCV genotype-I and genotype-II can be distinguished from the HCV genotype-III and genotype-IV.

Further, the present invention provides an antigen for measuring an HCV antibody, which enables the determination of the HCV antibody wherein the HCV genotype-I and genotype-II can be distinguished from the HCV genotype-III and genotype-IV; and further a cross-reaction or non-specific reaction is suppressed.

Further, the present invention provides a method of simply measuring an HCV antibody, wherein the HCV genotype-I and genotype-II can be distinguished from the HCV genotype-III and genotype-IV.

Further, the present invention provides a method of simply measuring an HCV antibody, wherein the HCV genotype-I and genotype-II can be distinguished from the HCV genotype-III and genotype-IV; and further a cross-reaction or non-specific reaction is suppressed.

Further, the present invention provides a method of measuring an HCV antibody at a low cost, wherein the HCV genotype-I and genotype-II can be distinguished from the HCV genotype-III and genotype-IV.

## Claims

1. An antigenic peptide compound, comprising a sequence which comprises at least six sequential amino acids and is included in an amino acid sequence represented by the following formula (1):
Arg-Lys-Thr-Ser-Glu-Arg-Ser-Gln-Pro-Arg-Gly-Arg-Arg-Gln-Pro-Ile-Pro-Lys-Ala-Arg-Arg-Pro-Glu-Gly-Arg-Thr-Trp-Ala-Gln-Pro ······ (1)

2. An antigenic peptide compound, comprising a sequence which comprises at least six sequential amino acids and is included in an amino acid sequence represented by the following formula (2):
Arg-Lys-Thr-Ser-Glu-Arg-Ser-Gln-Pro-Arg-Gly-Arg-Arg-Gln-Pro-Ile-Pro-Lys-Asp-Arg-Arg-Ser-Thr-Gly-Lys-Ser-Trp-Gly-Lys-Pro ······ (2)

3. An antigenic peptide compound according to Claim 1 or 2, which has been produced by using a chemical technique.

4. An antigenic peptide compound according to Claim 1 or 2, which has been produced by using a genetic engineering technique.

5. An immunological method of measuring an HCV antibody, comprising:
fixing the antigenic peptide according to Claim 1 or 2 to a carrier ;
binding an HCV antibody in a specimen to the peptide by utilizing an antigen-antibody reaction; and
binding a labeled ligand to the HCV antibody by utilizing an antigen-antibody reaction, thereby to measure the HCV antibody in the specimen.

6. An immunological method of measuring an HCV antibody according to Claim 5, wherein an enzyme-labeled ligand is used as the labeled ligand.

7. An immunological method of measuring an HCV antibody according to Claim 5, wherein a radioactive substance-labeled ligand is used as the labeled ligand.

8. An immunological method of measuring an HCV antibody according to Claim 5, wherein a fluorescent substance-labeled ligand is used as the labeled ligand.

9. An immunological method of measuring an HCV antibody according to Claim 5, wherein two or more kinds of antigenic peptides selected from the group consisting of the antigenic peptides according to Claim 1 or 2 are used so as to determine the serotype of HCV.

10. An immunological method of measuring an HCV antibody according to Claim 5, wherein two or more kinds of antigenic peptides corresponding to different regions of the HCV are used so as to determine the serotype of HCV, said two or more kinds of the antigenic peptides being elected from the group consisting of the antigenic peptides according to Claim 1 or 2 corresponding to the core region of HCV, and antigenic peptides corresponding to the NS-4 and NS-5 regions of HCV.
